Europäisches Patentamt

European Patent Office

Office européen des brevets

(11) Publication number: 0 391 716
A1

# EUROPEAN PATENT APPLICATION

(21) Application number: 90303669.7

(22) Date of filing: 05.04.90

(51) Int. Cl.5: C07C 263/06, C07C 265/12

(30) Priority: 06.04.89 US 334023
30.10.89 US 428567

(43) Date of publication of application:
10.10.90 Bulletin 90/41

(84) Designated Contracting States:
AT BE DE FR GB IT NL

(71) Applicant: ARCO CHEMICAL TECHNOLOGY INC.
3 Christina Centre Suite 902 201 N Walnut Street
Wilmington Delaware 19801(US)

(72) Inventor: Shawl, Edward T.
208 Martroy Lane
Wallingford, Pennsylvania 19086(US)
Inventor: Kesling, Haven S., Jr.
248 Friendship Road
Drexel Hill, Pennsylvania 19026(US)
Inventor: Liotta, Frank J., Jr.
105 Larchwood Court
Collegeville, Pennsylvania 19426(US)
Inventor: Zajacek, John G.
669 Clovelly Road
Devon, Pennsylvania 19333(US)

(74) Representative: Cropp, John Anthony David et al
MATHYS & SQUIRE 10 Fleet Street
London, EC4Y 1AY(GB)

(54) An integrated process for the preparation of aromatic mono- and polyisocyanates from aromatic amines.

(57) A multi-step process for the preparation of aromatic mono- and polyisocyanates in which an aromatic mono- or polyamine and isocyanic acid are reacted to form an aromatic urea which is then reacted with a dialkyl amine to give an aromatic dialkyl urea product. The aromatic dialkyl urea is thermally treated in an inert organic solvent in the presence of a reaction promoter to produce the isocyanate.

# AN INTEGRATED PROCESS FOR THE PREPARATION OF AROMATIC MONO- AND POLYISOCYANATES FROM AROMATIC AMINES

## FIELD OF THE INVENTION

The present invention relates to a multi-step process for the preparation of aromatic mono- and polyisocyanates. An aromatic mono- or polyamine and isocyanic acid are reacted to form an aromatic urea which is then reacted with a dialkyl amine to give an aromatic dialkyl urea product. The aromatic dialkyl urea is then thermally treated in an inert organic solvent in the presence of a reaction promoter to produce the isocyanate. The aromatic mono- and polyisocyanates produced by the instant process are of significant industrial importance, especially in the preparation of polyurethanes.

## BACKGROUND OF THE INVENTION

A number of processes have been reported for the preparation of mono- and disubstituted ureas and amines as in the preparation of, for example, the bis (diethylurea) of toluene by reacting toluene-2,4-diisocyanate with diethylamine described in Fr. Pat. No. 1,520,620.

An article by N. A. Ivanov entitled "Synthesis of Substituted Ureas and Thioureas and Their Thermal Stability", Chemistry Department of the Kalinin Agricultural Institute, describes the synthesis of thioureas, monosubstituted ureas and toluene-2,4-diamines.

German Democratic Republic Industrial Patent No. 228,544 related to the production of acyl isocyanates describes the synthesis of 1,1-diacyl-3,3-dialkylureas from, for example, 1,1-dimethylurea.

Czechoslovakian Patent No. 200,441 discloses a method for the preparation of aminophenylurea by reacting phenylenediamine with one mole of cyanic acid in the presence of sodium or potassium cyanate.

An article of Y. Shimonura et al entitled "Reactions of Isocyanic Acid with Various Reagents", Fukui Daigaku Kogakuba Kenkyu Hokoku, Vol. 31, No. 2, pp. 115, 1983 describes the reaction of 2-cyanoethylamine with isocyanic acid to give 2-cyanoethylurea. The synthesis of isocyanic acid from cyanuric acid by thermal decomposition is also set forth.

Applicants have found that tertiary amine hydrohalides such as pyridine hydrochloride, phosphorus pentoxide ($P_2O_5$), organic sulfonic acids and sulfonated aromatic ion exchange resins are all excellent promoters for the thermal decomposition of the intermediate aromatic dialkyl ureas of this invention to the corresponding isocyanate at relatively mild reaction temperatures and short residence times in an organic solvent.

Processes have been reported in the literature for the preparation of aromatic mono- and polyisocyanates by the vapor or solvent phase decomposition of substituted ureas using various promoters to help convert the urea groups to isocyanate groups.

The vapor phase production of aromatic isocyanates from symmetrical bis aryl ureas in the presence of hydrogen chloride, phosphorus pentoxide or zinc chloride was described by A. Hofmann in the Proc. Royal Soc., London, Vol. IX, p. 274-(1858). By heating a mixture of diphenyl urea with phosphorus pentoxide, zinc chloride or gaseous HCl, Hofmann distilled phenyl isocyanate overhead. No details of the experimental procedure are presented and the yield of isocyanate is not given.

A. Hofmann, Chemisch Berichte, Vol. 3, pp. 653-658(1870) described heating diphenyl urea in the presence of phosphorus pentoxide giving yields too low to be considered useful for the preparation of the isocyanate.

Subsequent work by Iwakura and Nagakubo reported in the Bulletin Tokyo Inst. Technol., Vol. 13, p. 25(1950) and Chemical Abstracts, Vol. 44, p. 3924e(1950) describes the preparation of an aromatic isocyanate (p-ethoxyphenylisocyanate) by heating a solution of a bis aryl urea such as bis (p-ethoxyphenyl) urea in the presence of hydrogen chloride gas.

The vapor phase decomposition of bis aryl ureas in the presence of hydrogen chloride promoter at temperatures of at least 350° C has been described by W. D. Bennet et al, Journ. Am. Chem. Soc., Vol. 75, p. 2101(1952) and Slocombe et al in U.S. Pat. No. 2,773,086. Yields are reported in the 60 to 70% range for the vapor phase reaction and only a 5% yield for liquid phase reaction. A carbamoyl chloride intermediate is formed.

The liquid phase decomposition of trisubstituted ureas to isocyanates has been described by van Landeghem et al, French Patent No. 1,473,821, February 13, 1967; C. J. Hearsey, U.S. Pat. No. 3,898,259, August 5, 1975 and Rosenthal et al, U.S. Pat. No. 3,936,484, February 3, 1976. van Landeghem shows thermal decomposition of trisubstituted ureas in an organic solvent having specified dielectric constants at 140° to 170° C with long reaction times of from 6 to 10 hours and modest yields of 60 to 75%. A variety of catalysts are shown but not exemplified or claimed, and include metal salts, such as acetates, stearates, and linoleates of manganese, zinc, cobalt, chromium and vanadium, tertiary amine bases, such as aliphatic, cycloaliphatic, aromatic and mixed tertiary amines, aliphatic heterocyclic amines such as N-methylpiperidine or N, N'-dimethylpiperidine as well as aromatic heterocyclic amines such as pyridine and pyrimidine. Other nitrogen compounds such as imidazole are indicated as being suitable. However, under the reaction conditions described, tertiary amines as shown by van Landeghem do not catalyze urea decomposition.

Rosenthal et al U.S. Pat. No. 3,936,484 discloses the thermal decomposition of di- and tri-substituted ureas to isocyanates at temperatures above 230°C in a solvent with short residence times and isocyanate yields of from 60 to 80%.

Hearsey (U.S. Pat. No. 3,898,259) describes the introduction of gaseous hydrogen chloride into the liquid phase urea decomposition reaction to give reduced reaction times with isocyanate yields of from 80 - 90%. An excess of gaseous HCl is employed at temperatures of from 100° to 200°C. A carbamoyl chloride by-product can be formed.

A. Hentschel et al U.S. Pat. No. 4,223,145, September 16, 1980 discloses the formation of an HCl adduct of a trisubstituted urea using, at most, a 10% excess of HCl. This adduct is then decomposed in a closed system at a temperature of from 80° to 180°C.

## SUMMARY OF THE INVENTION

This invention relates to a novel integrated multi-step process for the preparation of aromatic mono- and polyisocyanates from aromatic mono- or polyamines and isocyanic acid. Aromatic amines are reacted with isocyanic acid (HNCO) to convert the amino groups to urea groups (-NHCONH$_2$). The aromatic urea compounds are then reacted with a dialkyl amine such as diethylamine to produce an aromatic mono- or polydialkyl urea. The intermediate dialkyl urea, which is slurried in or dissolved in an inert organic solvent, is then thermally decomposed in the presence of a reaction promoter selected from tertiary amine hydrohalides, phosphorus pentoxide, organic sulfonic acids, perfluoroalkane sulfonic acid resins, and sulfonated aromatic ion exchange resins to produce the corresponding aromatic mono- or polyisocyanate.

It is an object of the present invention, therefore, to provide an improved multi-step process for the preparation of aromatic mono- and polyisocyanates from the urea reaction product of an aromatic amine and isocyanic acid which has been reacted with a dialkyl amine to give an aromatic dialkyl urea precursor to the corresponding isocyanate.

It is another object of this invention to provide an improved reaction (thermal decomposition) system for the conversion of the intermediate aromatic dialkyl urea to the corresponding aromatic isocyanate.

These and other objects and advantages of this invention will become apparent from the description of the invention which follows, and from the claims.

## DETAILED DESCRIPTION OF THE INVENTION

In accordance with this invention, an aromatic mono- or polyisocyanate having the general formula
Ar(NCO)n
wherein Ar is a mono-, di- or polyvalent aromatic radical which may be substituted with a halogen group, ether group, nitro group or an alkyl group having from 1 to 10 carbon atoms and n is an integer of from 1 to 3, is produced by a process which comprises the steps of

(a) reacting an aromatic amine having the formula
Ar(NH$_2$)n
wherein n and Ar are as described hereinabove, with isocyanic acid at a temperature of from about -30°C to about 200°C preferably from about -10°C to 100°C, in the presence of an inert organic solvent to convert the aromatic amine to an aromatic urea having the formula
Ar(NHCONH$_2$)n
wherein n and Ar are as described hereinabove;

(b) reacting said aromatic urea with a dialkyl amine having from 1 to 8 carbon atoms in the alkyl group, such as diethyl amine, at a temperature of from about 50° to about 200°C (more preferably, at a temperature of from about 90°C to 150°), in an inert organic solvent to produce an aromatic dialkyl urea having the general formula
Ar(NHCONR'R")n
wherein n and Ar are as described hereinabove and R' and R", which may be the same or different, are alkyl groups having from 1 to 8 carbon atoms;

(c) thermally decomposing said aromatic dialkyl urea dissolved in or slurried in an organic solvent or mixture of solvents, which are stable and substantially chemically inert to the components of the reaction system, in the presence of a reaction promoter selected from the group consisting of tertiary amine hydrohalides, phosphorus pentoxide, organic sulfonic acids and sulfonated aromatic ion exchange resins, at a temperature within the range of from about 50°C to about 220°C, preferably from about 90°C to about 150°C; and

(d) recovering the aromatic mono- or polyisocyanate.

The aromatic amines which are reacted with isocyanic acid in the process of the present invention may carry hydrogen atoms at the other ring positions, or they may be substituted by one or more groups such as an alkyl group having from 1 to 10 carbon atoms, a halogen atom, a nitro group or an ether group. Representative aromatic amines as hereinabove described include, for example, aniline, o-toluidine, m-toluidine, p-toluidine, 4-chloroaniline, 1,2-phenylenediamine, 4-chloro-1,2-phenylenediamine, 1,3-phenylenediamine, 1,4-phenylenediamine, 3,4-toluenediamine, 2,4-toluenediamine, 2,5-toluenediamine, 2-nitro-1,4-phenylenediamine, 4-methoxy-1,3-phenylenediamine, 2-ethoxy-1,3-phenylenediamine, 4,4′-diamino diphenyl, 2,4-diamino mesitylene, 1,3,5-triamino-2-methylbenzene, 1,8-diamino naphthalene, and the like.

The isocyanic acid employed in the process of the present invention may be produced or generated by known methods such as the pyrolysis of urea or cyanuric acid, reaction of cyanate salts such as sodium, potassium or silver cyanate and the like with an acid such as acetic or hydrochloric acid and the like. The isocyanic acid may be generated and used in situ, or it may be distilled away from its source and used in the process of the invention as a gas or dissolved in an appropriate solvent.

In the present process the molar ratio of the (-NH₂) groups of the aromatic amines to isocyanic acid is preferably at least about one to one. However, an excess of isocyanic acid of up to about 50% may be employed. Alternatively, an excess of up to about 30% (-NH₂) groups may be used and any unreacted or partially reacted aromatic amines separated from the aromatic urea produced and recycled. In the subsequent reaction employing the aromatic urea, the molar ratio of the dialkylamine reactant, such as dimethylamine, to the urea groups (-NHCONH₂) is preferably about one to one. However, an excess of dialkylamine of up to about a ten-fold excess may advantageously be employed to drive the reaction to completion. Unreacted dialkylamine may be easily recovered by distillation for recycle in the reaction.

The dialkylamines or mixtures thereof which may be employed in the process of the invention conform to the general formula $R'R''NH$ wherein $R'$ and $R''$ may be the same or different and are alkyl groups having independently from 1 to 8 carbon atoms. Representative dialkylamines include, for example, dimethylamine, diethylamine, methylethylamine, diisopropylamine, dicyclohexylamine, dibutylamine, and the like.

Solvents or mixtures of solvents which are stable and substantially chemically inert to the components of the reaction system are employed in each of the process steps of the present invention. Solvents having normal boiling points of from about 75°C to 200°C are preferred for use. Suitable solvents which may be employed include, for example, the aromatic hydrocarbons such as benzene, toluene, xylene, ethylbenzene, cumene, tetrahydronaphthalene as well as higher alkyl-substituted aromatic hydrocarbons; alkanes and substituted alkanes as well as cycloalkanes having from 5 to 20 carbon atoms such as, for example, n-hexane, n-heptane, octane, nonane, cyclohexane, dodecane, octadecane, 2-methyl-hexane, 2-ethylhexane, methyl-cyclohexane, cyclopentane and the like; halogenated or nitrated aromatic or aliphatic hydrocarbons such as, for example, methylene chloride, chloroform, carbon tetrachloride, 1,2-dichloroethane, chlorobenzene, trichloroethane, tetrachloroethane, dichlorobenzene, nitrobenzene, dinitrotoluenes and the like; aromatic or aliphatic ethers such as, for example, diphenylether and dibutylether and the like; tertiary amines such as, for example, pyridine, triethylamine, N-methylpyrrolidinone and the like. Certain ketones, esters, alcohols as well as water and highly polar solvents, such as sulfolane, dimethylsulfoxide, ethylene carbonate or propylene carbonate may also be used. It is not necessary that the aromatic amines, the reaction intermediates such as the aromatic ureas or the aromatic dialkyl ureas be completely miscible with the solvents at the concentrations employed. Advantage may be taken of differing solubilities of the reagents, intermediates and reaction products in the various solvents or mixture of solvents to separate the reaction components or to drive the reaction to increased product. The same solvent or mixture of solvents may be used throughout the reaction system or different solvents or solvent mixtures used in different steps of the process.

The intermediate aromatic urea obtained by the reaction of an aromatic amine with isocyanic acid may, if desirable, be isolated from the reaction system and further processed according to the process of the invention or it may simply be carried forward as a solution or a slurry without isolation for completion of the process to produce the desired isocyanate. In appropriate solvents, the intermediate aromatic urea may be essentially insoluble and the urea easily separated from unreacted aromatic amine or partly reacted amine urea by-products by conventional techniques such as filtration, centrifugation, and the like. The separated aromatic urea or the crude intermediate reaction product may be reacted with a dialkyl amine to produce the desired aromatic dialkyl urea. The dialkyl amine reactant may be added as a gas, a liquid, a solid or as a solution in solvent. Alternatively, the dialkyl amine may be added to the reactor along with the aromatic diamine. Ammonia generated in this part of the reaction is removed by any convenient means.

Referring to the general formulae set forth hereinabove Ar is preferably an aryl radical such as the mono-, di-, and trivalent radicals of benzene, toluene, naphthalene, diphenyl, terphenyl and the like. The aryl radicals may carry from 1 to 3 dialkyl urea substitutents and may have hydrogen atoms at the other ring positions or they may be substituted by one or more groups such as an alkyl group having from 1 to 10 carbon atoms, a halogen radical, a nitro group, an ether group, or other groups which are non-reactive with the isocyanates produced and other components of the reaction system.

The $R'$ and $R''$ of the aromatic dialkyl urea formula set forth hereinabove may be substituted or unsubstituted, mono-, di-, or trivalent radicals selected from saturated or mono- olefinic unsaturated straight or branched chain aliphatic or cycloaliphatic radicals optionally containing alkoxyalkyl radicals with one or more ether linkages, aryl radicals, or aralkyl radicals. These radicals may be substituted with groups which are non-reactive with the isocyanate produced by the process of the invention such as, for example, nitro or halo groups. Also included are cycloaliphatic and substituted cycloaliphatic radicals containing from 5 to 7 carbon atoms such as cyclopentyl, cyclohexyl and cycloheptyl radicals. Representative aromatic dialkyl urea compounds which may be employed in the process of the present invention include for example, N-phenyl-N', N'-dimethylurea, N-phenyl-N, N'-diethylurea, N-phenyl-N'-methyl-N'-ethylurea, N-phenyl-N',N'-dicyclohexylurea, 2,4-tolylene-(bis diethylurea), 2,6-tolylene-(bis dimethylurea), 2,4,6-tolylene-(tris dibutylurea), N-(2-chlorophenyl)-N',N'-(dicyclohexyl) urea and the like. The urea compounds above described are merely representative of a large number of aromatic dialkyl ureas falling within the general formula above which can be converted to isocyanates in the presence of the promoters of the present invention.

Representative aryl isocyanates which may be produced by the process of the present invention include, for example, phenyl isocyanate, 4-chlorophenyl and 2-fluorophenyl isocyanates, 3,4-dichlorophenyl isocyanate, m-phenylene diisocyanate, 2,4-toluene diisocyanate, 2,6-toluene diisocyanate, 2,4,6-toluene trisocyanate, 1,5-naphthalene diisocyanate and the like compounds.

The tertiary amine hydrohalide salts employed in the process of the present invention to promote thermal decomposition of the aromatic dialkyl ureas to the corresponding isocyanates may be prepared, for example, by reacting the tertiary amine selected with a hydrogen halide such as HCl. Salts of hydrogen fluoride, chloride, bromide or iodide may be used. The tertiary amines used to prepare the hydrohalide may have to the general formula $R,R',R''N$ wherein $R,R'$ and $R''$ are not hydrogen but may be an aliphatic radical having from 1 to 10 carbon atoms, a cycloaliphatic radical such as cyclopentyl, cyclohexyl and cycloheptyl radicals, an aromatic radical, or an aralkyl radical. Such radicals may be substituted with, for example, nitro or halo groups which are non-reactive with the isocyanate produced. Suitable amine salts include, for example, triethylamine hydrochloride, hydrobromide or hydrofluoride, trioctylamine hydrochloride, hydrobromide or hydrofluoride, N-methyl diethylamine hydrobromide or hydrochloride, N,N-diethylaniline hydrochloride and N,N-dimethylcyclohexylamine hydrochloride. Hydrohalide salts of heterocyclic tertiary amines and heterocyclic aromatic amines may also be employed as tertiary amine hydrohalide promoters. Representative salts of this type include, for example, N-methylpyrrolidine hydrochloride, pyridine hydrochloride or hydrobromide, lutidine hydrochloride or hydrobromide, 3-ethylpyridine hydrochloride, the hydrohalide salt of 1,4-diazabicyclo [2.2.2] octane, 4-chloro-pyridine hydrochloride, 4,4'-bipyridine dihydrochloride, quinoline hydrochloride or the like. Salts of amine oxides such as 2-chloropyridine N-oxide hydrochloride may also be used as tertiary amine hydrohalide promoters. In addition, the hydrohalide may be formed with an amine which may be part of a polymer such as polyvinyl pyridine or a resin prepared from tertiary amine groups attached to a styrene divinylbenzene polymer. The tertiary amine hydrohalide is generally employed in the process at a molar ratio of about one to one based on the urea groups. However, an excess of the tertiary amine hydrohalide promoter may be used.

The phosphorus pentoxide employed in the process of the present invention to promote thermal decomposition of the aromatic dialkyl ureas to the corresponding isocyanate is commercially available material produced by burning phosphorus in dry air. The phosphorus pentoxide promoter is generally employed in the process at a molar ratio of about one to one based on the urea groups. However, an excess of the phosphorus pentoxide promoter of up to three to one may be used. No advantage is generally gained by using higher amounts, which may lead to by-product formation.

The organic sulfonic acid employed in the process of the present invention to promote the thermal decomposition of the aromatic dialkyl ureas may be an alkane sulfonic acid or a halogenated alkane sulfonic acid having up to 10 carbon atoms in the alkyl group, or an aromatic sulfonic acid which may contain substitutents on the aromatic ring such as halogens, alkyl radicals, aromatic radicals, nitro groups and the like. The organic sulfonic acid may be in the form of an acidic sulfonated aromatic ion exchange resin such as, for example, a sulfonated styrene/divinyl-benzene copolymer (sold commercially, for example, as "Amberlyst 15" by Rohm & Haas Co. and having a bulk density of approximately 565 g./l, a hydrogen ion concentration of approximately 4.9 milliequivalents/g.dry, a surface area of from about 40 to 50 $M^2$/g. and an average pore diameter of from about 200 to 600 Angstrom units), or an acidic perfluoroalkane sulfonic acid resin such as "Nafion" (sold commercially, for example, by the Dupont Co. and having an equivalent weight of between about 110 and 1500, a hydrogen ion concentration of between about 0.7 - 1.0 milliequivalents/g.dry) which may be prepared, for example, by the polymerization of tetrafluoroethylene with a sulfonyl fluoride vinyl ether, followed by saponification with caustic to form the alkali metal salt and treatment with an acid to convert the salt to the sulfonic acid form. Mixtures of the sulfonic acid promoters may be employed but it is preferably to use a single acid promoter to simplify separation and recovery of the aromatic isocyanate produced. Representative organic sulfonic acid promoters suitable for use in the process of this invention include, for example, methane, ethane, butane, hexane sulfonic acids, and the like, trifluoromethane sulfonic acid, benzene sulfonic acid, 1,3-benzene disulfonic acid, p-toluene sulfonic acid, naphthalene sulfonic acid, 4-chloro-3-nitrobenzene sulfonic acid, 3-nitrobenzene sulfonic acid, and the like, as well as the acidic ion exchange resins which include the "Amberlyst 15" and "Nafion" resins described hereinabove and also include the "Dowex 50" (Dow Chemical), "AG50W" (Bio-Rad), and "Amberlyst XN-1010", "Amberlite IR-118", "Amberlite IR-120", "Amberlite IRP-69" (Rohm and Haas) resin materials. The ion exchange resins may be supplied commercially in the hydrogen ion form or the salt form such as the sodium or potassium salt. The salt can readily be converted to the active hydrogen ion form by, for example, treating with aqueous hydrochloric acid, washing with water to a constant pH in the range of 5.5 to 7 and then drying to remove residual water.

The organic sulfonic acid promoter is generally employed in the process of the instant invention at a molar ratio of about one to one based on the urea groups to sulfonic acid groups. No advantage is normally gained by using large excess amounts, which may lead to by-product formation.

The process of the present invention can be suitably carried out by combining the aromatic dialkyl urea with a solvent or a mixture of solvents comprising the reaction medium. The urea may be soluble in the solvent or solvents or soluble at reaction temperatures or the urea may be in the form of a slurry. Reactions using an acidic ion exchange resin promoter may also be carried out in a fixed bed of resin by passing a solvent solution of the urea through a bed maintained at the desired reaction temperature. A general procedure for carrying out step (a) of the process is to charge the aromatic amine together with a solvent into the reaction vessel. The isocyanic acid is introduced into the reactor as a gas, optionally diluted with an inert gas, as a liquid, or as a solution in an appropriate solvent. Alternatively, the isocyanic acid can be charged to the reactor first together with a solvent and the aromatic amine then added as a liquid, a solid, a solution in a suitable solvent or as a slurry in a suitable inert liquid. The reaction vessel is heated or cooled as necessary to provide the desired reaction temperature for the appropriate period. Heating and/or cooling means may be employed interior or exterior of the reaction vessel to maintain temperature within desired ranges. The reaction product may be recovered by standard filtration and/or distillation procedure, if desired, or the mixture may be simply used as is in the next step of the process.

In the process of the present invention, the reaction of the aromatic amine with isocyanic acid will proceed at temperatures of from about -30°C to about 200°C, preferably from about -10°C to about 100°C. Reaction time is dependent on the temperature but will generally range between about two minutes to several hours. The reaction of the intermediate aromatic urea and the dialkyl amines will proceed at temperatures of from about 50°C to about 200°C, preferably from about 90°C to to about 150°C. The reaction time depends on the temperature but will generally range between about 30 minutes to about 8 hours.

The process of the present invention may be carried out as a batch, semi-continuous or continuous process and the order of addition of the materials and reactants may be varied to suit the particular apparatus, reactants, and promoter employed.

The reaction of the invention may be carried out in any suitable reactor which is equipped with a means for temperature control and agitation. Heating and/or cooling means may be employed interior or exterior of the reaction vessel to maintain temperature within the desired range.

As indicated hereinabove, the thermal decomposition of the aromatic dialkyl urea is carried out at temperatures of from about 50°C to about 220°C, preferably from about 90°C to 150°C. Reaction time is dependent on decomposition temperature but will generally range between about 5 minutes and 10 hours.

The reaction steps of this invention are generally carried out at atmospheric pressure. Depending on the boiling points of the solvents and the isocyanate product, any or all of the steps may be carried out at super-atmospheric pressures.

The isocyanate formed may be recovered by filtration, by distillation of either the solvent or the isocyanate, whichever is lower boiling, or by other known methods. The particular recovery method chosen will depend on the solvent employed and the isocyanate produced.

The present invention is more fully illustrated by the following examples, which include particular features of the invention. However, the examples are not to be construed as limiting the invention in any way, it being understood that numerous variations are possible without departing from the spirit and scope of the invention.

## EXAMPLE 1

A solution of 0.65g, 5.30 mmol, 2,4-toluenediamine in 61.0g of pyridine was charged to a 250 mL Erlenmeyer flask. The solution was stirred with a magnetic stirrer while 13.2g of a 4.60 weight percent solution of isocyanic acid in toluene containing 0.61g, 14.2 mmol, of isocyanic acid was added over one minute at room temperature. Solids began to precipitate immediately on addition of the isocyanic acid solution. Analysis of the solids and liquid phase by high pressure liquid chromatography (HPLC) showed 97% conversion of the 2,4- toluenediamine with 85% selectivity to the 2,4-bis urea of toluenediamine as solids and 13% selectivity to amine ureas which remained in solution in the pyridine solvent. The bis urea solids (0.94g) were separated by filtration and suspended in 50g 1,2-dichlorobenzene and added to a 300 ml, 3-neck round bottom flask fitted with a magnetic stirrer, thermometer, and a condenser. Diethylamine, 2.90g or 39.7 mmol, was added to the flask and the mixture was heated at 130° C for two hours. Analysis of the product by HPLC showed complete conversion of the 2,4-bis urea of toluenediamine to the bis (diethylurea) of 2,4-toluenediamine.

A mixture of 32g of the 2,4-tolylene (bisdiethylurea) and 35g pyridine hydrochloride in 500g o-xylene was heated at reflux for 90 minutes in a 1 liter fluted flask equipped with a mechanical stirrer and condenser. The product was cooled and then the organic phase was decanted from the salts. Conversion of the tolylene (bisdiethylurea) was 99% with selectivity of 95% to 2,4-toluene diisocyanate and 3% to the monoisocyanate monodiethylurea derivatives. Toluene diisocyanate was isolated by distillation from the xylene solution. The isolated product weighted 16g corresponding to a 92% overall yield.

## EXAMPLE 2

A solution of 0.80g of 2,4-toluenediamine in 50g of 1,2-dichloroethane was charged to a 250 mL Erlenmeyer flask containing 0.64g (14.9 mmol) isocyanic acid dissolved in 8g dichloromethane. The mixture was stirred for one hour at room temperature (approximately 25° C). Solids precipitated immediately on addition of the toluenediamine solution. Analysis of the recovered solids, 1.41g, and the solution by HPLC analysis showed 99% conversion of the toluenediamine with 98% selectivity to the bis urea of 2,4-toluenediamine. The solids were suspended in 50g 1,2-dichlorobenzene in a 300 mL, 3-neck, round bottom flask equipped with a mechanical stirrer, condenser and thermometer. Diethylamine, 4.8g or 66 mmol was added and the mixture was heated for two hours. Initially the temperature was limited to 120° C by reflux of the diethylamine, but as the diethylamine was consumed, the temperature was gradually increased to 150° C.

The product, the bis (diethylurea) of 2,4-toluenediamine and which was soluble in dichlorobenzene, was isolated by distilling off the solvent and unreacted diethylamine using a rotary evaporator. The solid recovered, 1.97g, was analyzed by HPLC as 97% bis (diethylurea) of 2,4-toluenediamine and corresponded to a 91% overall yield of the bis (diethylurea) based on starting toluenediamine.

A mixture of 5.26g, 16.4 mmol, of the 2,4-toluene bis (diethylurea) in 505g toluene was added to a liter, 3-neck, fluted, bottom flask equipped with a bottom take-off, mechanical stirrer, condenser and thermocouple for measuring reaction temperature. The mixture was heated to gentle reflux at 110° and then 3.49g, 36.3 mmol, of methane sulfonic acid was added. The mixture was stirred 15 min. at 110° C and then the mixture was allowed to settle at 60° C. The salt separated as a liquid bottom phase and was drawn off through the bottom take-off. The toluene phase was analyzed directly by infrared analysis for 2,4-toluene diisocyanate (TDI) by comparing the isocyanate band in the product with that in known standards of pure TDI in toluene. The yield of TDI was 98%. Another sample from the pot was treated with ethanol to convert the TDI to its ethyl carbamate derivative and this was analyzed by high pressure liquid chromatography (HPLC). This analysis, confirming the infrared analysis, showed 100% conversion of the starting toluene bis (diethylurea) with 98% selectivity to TDI.

## EXAMPLE 3

Isocyanic aid, generated by the reaction of sodium cyanate with hydrogen chloride, was distilled away from the sodium cyanate and carried as a gas diluted with nitrogen into a 300 mL, 2-neck round bottom flask equipped with a magnetic stirrer, condenser and gas inlet tube containing 1.20g, 10 mmol, of 2,4-toluenediamine in 100g o-dichlorobenzene. The reaction was held at -10°C by a refrigerated bath. Solids precipitated in the flask. After an excess of isocyanic acid had been added, the reaction was stirred for fifteen minutes and then 1.8g or 40 mmol of dimethylamine was added and the mixture kept at 65° for 8 hours. Analysis of the product by HPLC showed an overall yield of 90% of the 2,4-bis (dimethylurea) of toluenediamine.

2.6g of the 2,4-bis (dimethylurea) of toluenediamine in 100g mesitylene was added to a 1 liter reaction flask fitted with a mechanical stirrer, condenser and a thermocouple for measuring temperature. A nitrogen atmosphere was maintained over the reaction mixture, the mixture was heated to 155°C and a 1:1 molar ratio of phosphorus pentoxide based on the urea groups added to the reactor. Conversion of the urea was 66% with a 83% selectivity to the toluene diisocyanate.

## EXAMPLE 4

To a 200 mL, 3-neck reaction flask with a mechanical stirrer, condenser and thermometer was added an 80/20 mixture of 2,4-, 2,6-toluenediamine in 100g of diphenylether along with 1.00g of isocyanic acid in 10g diphenylether and the mixture heated to 150°C for 10 minutes. Diethylamine (15g) was added to the mixture which was then heated to 100°C for 4 hours. Yield of the bis (diethylurea) of 2,4- and 2,6-toluenediamine was 96%.

5.04g of the bis (diethylurea) of 2,4-, 2,6-toluenediamine prepared above was combined with 10g of "Amberlyst" 15 and 60mL of toluene in a round bottom flask and then heated at 10°C for 4 hours under a nitrogen atmosphere. The mixture was cooled to room temperature and the solid resin removed by filtration. Analysis of the solution by HPLC after addition of ethanol to convert the TDI to its ethyl carbamate derivative showed an 86% yield of the respective toluene diisocyanates.

## EXAMPLE 5

3.2g of the 2,4-tolylene (bis diethylurea) of Example 1 was added to a 3-neck round bottom reaction flask along with 3.5g of p-toluene sulfonic acid and 100g diphenylether. The mixture was heated to 130°C for 1 hour. Conversion of the urea was analyzed to be 95% with a selectivity to the 2,4-toluene diisocyanate of 89%.

## EXAMPLE 6

The procedure of Example 5 was repeated using 5.2g of 2,4-tolylene bis (dibutylurea), 100g toluene and 6.0g trifluoromethane sulfonic acid. The mixture was heated to 90°C for 10 minutes and gave a selectivity to the 2,4-toluene diisocyanate of 97%.

## EXAMPLE 7

This example illustrates the preparation of a monoisocyanate from an aromatic amine in accordance with the integrated process of this invention; a single solvent is used for each step of the process.

A solution of 2.14g (30 mmol) m-toluidine in 50g tetrachloroethane is charged to a 300 mL rounded bottom flask fitted with a magnetic stirrer, condenser, and a thermocouple for measuring reaction temperature and containing 1.29g (30 mmol) isocyanic acid dissolved in about 100 mL tetrachloroethane. The aromatic urea product precipitates as a solid immediately upon addition of the m-toluidine solution. The mixture is stirred for 1 hour at 25°C, treated with 3.88g (30 mmol) dibutylamine, and then heated at 130°C for 2 hours to form the aromatic dibutyl urea. Triethylamine hydrochloride promoter (2.75g; 20 mmol) is added and the resulting mixture heated at reflux for 45 minutes. 3-Toluene isocyanate is isolated by fractional distillation from the tetrachloroethane solution. The overall yield of isocyanate is expected to be greater than 90 percent.

**Claims**

1. A process for the preparation of an aromatic mono- or polyisocyanate having the general formula

Ar(NCO)n

wherein Ar is a mono-, di- or polyvalent aromatic radical which may be substituted with one or more substituents selected from halogen groups, nitro groups and alkyl groups having from 1 to 10 carbon atoms, and n is an integer of from 1 to 3, which comprises the steps of

(a) reacting an aromatic amine having the formula

Ar(NH₂)n

wherein Ar and n are as hereinabove described, with isocyanic acid at a temperature of from about -30°C to about 200°C in the presence of an inert organic solvent to convert the aromatic amine to an aromatic urea having the formula

Ar(NHCONH₂)n

wherein Ar and n are as hereinabove described;

(b) reacting said aromatic urea with a dialkyl amine having from 1 to 8 carbon atoms in each alkyl group at a temperature of from about 50°C to about 200°C in an inert organic solvent to produce an aromatic dialkyl urea having the general formula

Ar(NHCONR′R″)n

wherein Ar and n are as hereinabove described and R′ and R″, which may be the same or different, are alkyl groups having from 1 to 8 carbon atoms;

(c) thermally decomposing said aromatic dialkyl urea, dissolved or slurried in an inert organic solvent, in the presence of a reaction promoter selected from the group consisting of tertiary amine hydrohalides, phosphorus pentoxide, organic sulfonic acids, perfluoroalkane sulfonic acid resins, and sulfonated aromatic ion exchange resins at a temperature of from about 50°C to about 200°C to form the aromatic mono- or polyisocyanate; and

(d) recovering the aromatic mono- or polyisocyanate.

2. A process according to Claim 1 wherein the temperature of reacting the aromatic amine with isocyanic acid is in the range of from about -10°C to 100°C.

3. A process according to Claim 1 or Claim 2 wherein the temperature of reacting the aromatic urea with the dialkyl amine is in the range of from about 90°C to 150°C.

4. A process according to any one of Claims 1 to 3 wherein the aromatic amine is selected from 2,4-diaminotoluene, 2,6-diaminotoluene and mixtures of 2,4- and 2,6-diaminotoluene.

5. A process according to any one of Claim 1 to 4 wherein the dialkyl amine is selected from dimethyl amine, diethyl amine, dibutyl amine and diisopropyl amine.

6. A process according to any one of Claims 1 to 5 wherein the temperature of thermally decomposing the aromatic dialkyl urea is in the range of from about 90°C to 150°C.

7. A process according to any one of Claims 1 to 6 wherein the aromatic dialkyl urea is selected from 2,4-tolylene-bis (diethylurea), 2,6-tolylene-bis (diethylurea), 2,4-tolylene-bis (dimethylurea), 2,6-tolylene-bis (dimethylurea), 2,4-tolylene-bis (dibutylurea), 2,6-tolylene-bis (dibutylurea), and mixtures of 2,4 and 2,6-tolylene bis (diethylurea).

8. A process according to any one of Claims 1 to 7 wherein the reaction promoter is a tertiary amine hydrohalide selected from pyridine hydrochloride, pyridine hydrobromide, triethylamine hydrochloride and trimethylamine hydrochloride.

9. A process according to any of of Claims 1 to 8 wherein the inert organic solvent is selected from aromatic hydrocarbons, alkyl-, halo-, or nitro-substituted aromatic hydrocarbons, alkanes and cycloalkanes having from 5 to 20 carbon atoms, halo- and nitro-substituted aliphatic hydrocarbons, aromatic ethers, aliphatic ethers and mixtures thereof.

10. A process according to any one of Claims 1 to 9 wherein the organic sulfonic acid is selected from methane sulfonic acid, p-toluene sulfonic acid, ethane sulfonic acid and trifluoromethane sulfonic acid.

11. A process according to any one of Claims 1 to 10 wherein the molar ratio of the reaction promoter and urea groups is about one to one.

12. A process for the preparation of a tolylene diisocyanate which comprises the steps of

(a) reacting a toluenediamine with isocyanic acid at a temperature of from about -10°C to 100°C in the presence of an inert organic solvent to convert the toluenediamine to an aromatic bis urea;

(b) reacting said aromatic bis urea with diethylamine in an inert organic solvent at a temperature of from about 90°C to 150°C to produce an aromatic bis diethylurea;

(c) thermally decomposing said aromatic bis diethylurea, dissolved or slurried in an organic solvent, in the presence of a reaction promoter selected from pyridine hydrochloride, phosphorus pentoxide, methane sulfonic acid, sulfonated styrene/divinylbenzene copolymer resin and perfluoroalkane sulfonic acid resin at a temperature of from about 90°C to about 150°C to form the tolylene diisocyanate; and

(d) recovering the tolylene diisocyanate.

13. The process of Claim 12 wherein the tolylene diisocyanate is selected from 2,4-tolylene diisocyanate, 2,6-tolylene diisocyanate, and mixtures thereof.

14. A process according to any one of Claims 1 to 13 wherein the inert organic solvent is selected from toluene, 1,2-dichlorobenzene, o-xylene, 1,2-dichloroethane, mesitylene, and diphenyl ether.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,P | EP-A-0 333 350 (ARCO CHEMICAL TECHNOLOGY INC.)<br>* claim 1 *<br>--- | 1 | C 07 C 263/06<br>C 07 C 265/12 |
| Y,P | US-A-4 871 871 (E.T. SHAWL et al.)<br>* claim 1 *<br>--- | 1 | |
| A | DE-A-2 837 341 (AKZO GMBH)<br>* claim 1 *<br>--- | 1 | |
| A | DE-A-2 225 365 (QUIMCO GMBH)<br>* page 5, last paragraph; claim 1 *<br>----- | 1 | |

|  |  |
|---|---|
|  | TECHNICAL FIELDS SEARCHED (Int. Cl.5)<br><br>C 07 C 263/06<br>C 07 C 265/12 |

The present search report has been drawn up for all claims

| Place of search | Date of completion of the search | Examiner |
|---|---|---|
| BERLIN | 05-07-1990 | KAPTEYN H G |